# EUROPEAN PATENT SPECIFICATION

(11) **EP 4 481 039 B1**
(45) Date of publication and mention of the grant of the patent: **25.11.2026**
(21) Application number: 22927426.1
(22) Date of filing: 11.07.2022
(51) Int. Cl.: C12N 9/04, C12N 15/52, C12P 13/22

(54) **NOVEL ZINC-BINDING DEHYDROGENASE VARIANT AND METHOD FOR PRODUCING L-AROMATIC AMINO ACID USING SAME**
NEUE ZINKBINDENDE DEHYDROGENASEVARIANTE UND VERFAHREN ZUR HERSTELLUNG VON L-AROMATISCHER AMINOSÄURE DAMIT
NOUVEAU VARIANT DE DÉSHYDROGÉNASE DE LIAISON AU ZINC ET PROCÉDÉ DE PRODUCTION D'ACIDE AMINÉ L-AROMATIQUE L'UTILISANT

(30) Priority: 16.02.2022 KR 20220020010
(43) Date of publication of application: 25.12.2024
(73) Proprietor: Daesang Corporation, Seoul 02586 (KR)
(72) Inventor: SHIN, Won Joo, Bucheon-si Gyeonggi-do 14506 (KR); PARK, Jae Eun, Incheon 21125 (KR); KIM, Yong Soo, Yongin-si Gyeonggi-do 17117 (KR); JO, Young Il, Seoul 04995 (KR)
(74) Representative: Ipsilon
(86) International application number: PCT/KR2022/010045
(87) International publication number: WO 2023/158030

(56) References cited:
- KR-A- 20060 127 990
- KR-B1- 101 947 959
- US-A1- 2016 333 325
- US-A1- 2021 324 430
- GU PENGFEI; KANG JUNHUA; YANG FAN; WANG QIAN; LIANG QUANFENG; QI QINGSHENG: "The improvedl-tryptophan production in recombinantEscherichia coliby expressing the polyhydroxybutyrate synthesis pathway", APPLIED MICROBIOLOGY AND BIOTECHNOLOGY, SPRINGER BERLIN HEIDELBERG, BERLIN/HEIDELBERG, vol. 97, no. 9, 16 January 2013 (2013-01-16), Berlin/Heidelberg, pages 4121 - 4127, XP035328824, ISSN: 0175-7598, DOI: 10.1007/s00253-012-4665-0
- VANHOVE MATHIEU, RECHTLESS ADAM C., SICARD ANNE, RIEUX ADRIEN, COLETTA-FILHO HELVECIO D., LA FUENTE LEONARDO, STENGER DRAKE C., AL: "Zinc Toxicity and Iron-Sulfur Cluster Biogenesis in Escherichia coli. Applied and Environmental Microbiology.", APPLIED AND ENVIRONMENTAL MICROBIOLOGY, vol. 85, no. 9, 1 May 2019 (2019-05-01), pages e01967 - 18, XP093085165, DOI: 10.1128/AEM
- DATABASE Protein 16 December 2020 (2020-12-16), ANONYMOUS : "MULTISPECIES: zinc-binding dehydrogenase [Enterobacteriaceae] ", XP093085166, retrieved from NCBI Database accession no. WP_000853247.1

## Description

### Technical Field

The present invention relates to a novel zinc-binding dehydrogenase variant and a method of producing an L-aromatic amino acid using the same.

### Background Art

Amino acids are classified into hydrophobic, hydrophilic, basic, and acidic amino acids, depending on the nature of their side chains, and among them, amino acids having a benzene ring are called aromatic amino acids. Aromatic amino acids include phenylalanine, tyrosine, and tryptophan. Phenylalanine and tryptophan are essential amino acids that are not synthesized *in vivo* and correspond to high value-added industries that form a global market worth $300 billion annually.

For the production of aromatic amino acids, either naturally occurring wild-type strains or mutant strains modified to have an increased ability to produce amino acids may be used. In recent years, in order to improve the efficiency of production of aromatic amino acids, there has been development of a variety of recombinant strains or mutant strains having excellent L-aromatic amino acid productivity by applying genetic recombination technology to microorganisms such as *Escherichia coli* and *Corynebacterium,* which are widely used in the production of useful substances such as L-amino acids, and methods of producing L-aromatic amino acids using the same. In particular, there have been attempts to increase the production of L-aromatic amino acids by targeting genes such as enzymes, transcription factors and transport proteins, which are involved in the biosynthetic pathways of L-aromatic amino acids, or by inducing mutations in promoters that regulate the expression of these genes. However, there are dozens of types of proteins such as enzymes, transcription factors and transport proteins, which are involved directly or indirectly in the production of L-aromatic amino acids, and thus much research is still needed on the increase in L-aromatic amino acid productivity by changes in the activity of these proteins.

### [Prior Art Documents]

### [Patent Documents]

Korean Patent No. 10-1830002
The document Vanhove Mathieu, Rechtless Adam C., Sicard Anne, Rieux Adrien, Coletta-Filho Helvecio D., La Fuente Leonardo, Stenger Drake C., Al: "Zinc Toxicity and Iron-Sulfur Cluster Biogenesis in Escherichia coli. Applied and Environmental Microbiology.",Applied and Environmental Microbiology, vol. 85, no. 9, 1 May 2019 (2019-05-01), pages e01967-18,discloses zinc-binding dehydrogenases from Enterobacteriaceae. It describes the identification and characterization of multispecies zinc-binding dehydrogenase enzymes, including their amino acid sequences and zinc-binding properties. The document provides information relevant to the structure and function of these dehydrogenases, which are involved in various metabolic pathways in Enterobacteriaceae. **DISCLOSURE**

### Technical Problem

An object of the present invention is to provide a novel zinc-binding dehydrogenase variant.

Another object of the present invention is to provide a polynucleotide encoding the variant.

Still another object of the present invention is to provide a transformant comprising the variant or the polynucleotide.

Yet another object of the present invention is to provide a method of producing an L-aromatic amino acid using the transformant.

### Technical Solution

One aspect of the present invention provides a zinc-binding dehydrogenase variant consisting of the amino acid sequence of SEQ ID NO: 1 in which glycine at position 102 in the amino acid sequence of SEQ ID NO: 3 is substituted with arginine.

The "zinc-binding dehydrogenase" as used in the present invention is a type of zinc-binding oxidoreductase that exhibits redox activity in the presence of NAD or NADP. The zinc-binding dehydrogenase may be a gene encoding zinc binding dehydrogenase or a sequence having substantial identity thereto. As used herein, "substantial identity" means that, when each gene sequence, i.e., a base sequence or nucleotide sequence, and any other nucleotide sequence are aligned to correspond to each other as much as possible and analyzed, the other nucleotide sequence has a sequence homology of at least 70%, at least 80%, at least 90%, or at least 98% with each nucleotide sequence.

The zinc-binding dehydrogenase in the present invention is encoded by the yggP gene and comprises the amino acid sequence of SEQ ID NO: 3.

According to one embodiment of the present invention, the amino acid sequence of SEQ ID NO: 3 may be derived from wild-type *E. coli.*

As used in the present invention, the term "variant" refers to a polypeptide which is obtained by conservative substitution and/or modification of one or more amino acids at the N-terminus, C-terminus of and/or within the amino acid sequence of a specific protein and has an amino acid sequence different from that of the protein, but retains functions or properties of the protein. As used herein, the term "conservative substitution" means replacing one amino acid with another amino acid having similar structural and/or chemical properties. The conservative substitution may have little or no impact on the activity of the protein or polypeptide. In addition, some variants include those in which one or more portions, such as an N-terminal leader sequence or transmembrane domain, have been removed, or those in which a portion has been removed from the N- and/or C-terminus of a mature protein. The ability of the variant may be increased, unchanged, or reduced compared to that of the polypeptide before modification. In the present invention, the term "variant" may be used interchangeably with terms such as variant type, modification, variant polypeptide, mutated protein, mutation, and the like.

The variant in the present invention may be a zinc-binding dehydrogenase variant consisting of the amino acid sequence of SEQ ID NO: 1 in which the amino acid glycine at position 102 in the amino acid sequence of SEQ ID NO: 3 is substituted with arginine.

Another aspect of the present invention provides a polynucleotide encoding the zinc-binding dehydrogenase variant.

As used in the present invention, the term "polynucleotide" refers to a DNA or RNA strand having a certain length or more, which is a long-chain polymer of nucleotides formed by linking nucleotide monomers via covalent bonds. More specifically, the term "polynucleotide" refers to a polynucleotide fragment encoding the variant.

The polynucleotide may comprise a nucleotide sequence encoding the amino acid sequence of SEQ ID NO: 1.

According to one embodiment of the present invention, the polynucleotide may comprise the nucleotide sequence represented by SEQ ID NO: 2.

Still another aspect of the present invention provides a vector comprising a polynucleotide encoding the zinc-binding dehydrogenase variant.

Yet another aspect of the present invention provides a transformant comprising the zinc-binding dehydrogenase variant or the polynucleotide.

As used in the present invention, the term "vector" refers to any type of nucleic acid sequence transfer structure that is used as a means for transferring and expressing a gene of interest in a host cell. Unless otherwise specified, the term "vector" may mean one allowing the nucleic acid sequence contained therein to be expressed after insertion into the host cell genome and/or one allowing the nucleic acid sequence to be expressed independently. This vector comprises essential regulatory elements operably linked so that an inserted gene can be expressed. As used herein, the term "operably linked" means that a gene of interest and sequences for regulating expression of the gene are functionally linked together in a manner enabling gene expression, and the term "regulatory elements" include a promoter for initiating transcription, any operator sequence for regulating transcription, a sequence encoding suitable mRNA ribosome-binding sites, and a sequence for regulating termination of transcription and translation.

The vector that is used in the present invention is not particularly limited as long as it may replicate in a host cell, and any vector known in the art may be used. Examples of the vector include a natural or recombinant plasmid, cosmid, virus and bacteriophage. Examples of a phage vector or cosmid vector include, but are not limited to, pWE15, M13, λMBL3, λMBL4, λIXII, λASHII, λAPII, λt10, λt11, Charon4A, and Charon21A, and examples of a plasmid vector include, but are not limited to, pBR series, pUC series, pBluescriptII series, pGEM series, pTZ series, pCL series, and pET series.

The vector may typically be constructed as a vector for cloning or as a vector for expression. The vector for expression may be a conventional vector that is used in the art to express a foreign gene or protein in a plant, animal, or microorganism, and may be constructed through various methods known in the art.

The recombinant vector may be constructed to use a prokaryotic or eukaryotic cell as a host. For example, when the vector used is an expression vector and uses a prokaryotic cell as a host, the vector generally comprises a strong promoter capable of promoting transcription (e.g., pLλ promoter, CMV promoter, trp promoter, lac promoter, tac promoter, T7 promoter, etc.), a ribosome binding site for initiation of translation, and a transcription/translation termination sequence. When a eukaryotic cell is used as a host, the vector comprises a replication origin operating in the eukaryotic cell, such as an f1 replication origin, an SV40 replication origin, a pMB1 replication origin, an adeno replication origin, an AAV replication origin, or a BBV replication origin, without being limited thereto. In addition, the recombinant vector may comprise a promoter derived from the genome of a mammalian cell (e.g., metallothionein promoter) or a promoter derived from a mammalian virus (e.g., adenovirus late promoter, vaccinia virus 7.5K promoter, SV40 promoter, cytomegalovirus promoter, HSV-tk promoter, etc.), and generally has a polyadenylation sequence as a transcription termination sequence.

The recombinant vector may comprise a selection marker. The selection marker serves to select a transformant (host cell) transformed with the vector, and since only cells expressing the selection marker can survive in the medium treated with the selection marker, it is possible to select transformed cells. Representative examples of the selection marker include, but are not limited to, kanamycin, streptomycin, and chloramphenicol.

The transformant may be produced by inserting the recombinant vector into a host cell, and the transformant may be obtained by introducing the recombinant vector into an appropriate host cell. The host cell is a cell capable of stably and continuously cloning or expressing the expression vector, and any host cell known in the art may be used.

When the vector is transformed into prokaryotic cells to generate recombinant microorganisms, examples of host cells that may be used include, but are not limited to, *E*. *coli* sp. strains such as *E. coli* JM109, *E. coli* BL21, *E*. *coli* RR1, *E. coli* LE392, *E. coli* B, *E. coli* X 1776, *E. coli* W3110, and *E. coli* XL1-Blue, *Bacillus* sp. strains such as *Bacillus subtilis,* and *Bacillus thuringiensis,* and various Enterobacteriaceae strains such as *Salmonella typhimurium, Serratia marcescens,* and *Pseudomonas* species.

When the vector is transformed into eukaryotic cells to generate recombinant microorganisms, examples of host cells that may be used include, but are not limited to, yeast (e.g., *Saccharomyces cerevisiae*), insect cells, plant cells and animal cells, such as Sp2/0, CHO K1, CHO DG44, PER.C6, W138, BHK, COS7, 293, HepG2, Huh7, 3T3, RIN, and MDCK cell lines.

As used in the present invention, the term "transformation" refers to a phenomenon in which external DNA is introduced into a host cell, thereby artificially causing genetic changes, and the term "transformant" refers to a host cell into which external DNA has been introduced and in which the expression of the gene of interest is stably maintained.

The transformation may be performed using a suitable vector introduction technique selected depending on the host cell, so that the gene of interest or a recombinant vector comprising the same may be expressed in the host cell. For example, introduction of the vector may be performed by electroporation, heat-shock, calcium phosphate (CaPO₄) precipitation, calcium chloride (CaCl₂) precipitation, microinjection, polyethylene glycol (PEG) method, DEAE-dextran method, cationic liposome method, lithium acetate-DMSO method, or any combination thereof, without being limited thereto. As long as the transformed gene may be expressed in the host cell, it may be integrated into and placed in the chromosome of the host cell, or it may exist extrachromosomally, without being limited thereto.

The transformant may include a cell transfected, transformed, or infected with the recombinant vector of the present disclosure *in vivo* or *in vitro,* and may be used in the same sense as a recombinant host cell, a recombinant cell, or a recombinant microorganism.

According to one embodiment of the present invention, the transformant may be an *Escherichia* sp. strain.

Examples of the *Escherichia* sp. strain include, but are not limited to, *Escherichia coli, Escherichia albertii, Escherichia blattae, Escherichia fergusonii, Escherichia hermannii, Escherichia vulneris,* and the like.

The transformant in the present invention may be a strain either comprising the above-described zinc-binding dehydrogenase variant or a polynucleotide encoding the same or comprising the vector comprising the same, a strain expressing the zinc-binding dehydrogenase variant or the polynucleotide, or a strain having activity for the zinc-binding dehydrogenase variant, without being limited thereto.

According to one embodiment of the present invention, the transformant may have the ability to produce an L-aromatic amino acid.

The transformant may naturally have the ability to produce an L-aromatic amino acid or may be one artificially endowed with the ability to produce an L-aromatic amino acid.

According to one embodiment of the present invention, the transformant may have an increased ability to produce an L-aromatic amino acid, due to a change in the activity of zinc-binding dehydrogenase.

As used in the present invention, the term "increased ability to produce" means that L-aromatic amino acid productivity has increased compared to that of the parent strain. As used herein, the term "parent strain" refers to a wild-type strain or mutant strain to be mutated, and includes a strain that is to be mutated directly or to be transformed with a recombinant vector or the like. In the present invention, the parent strain may be a wild-type *Escherichia* sp. strain or an *Escherichia* sp. strain mutated from the wild-type strain.

The transformant according to the present invention exhibits an increased ability to produce an L-aromatic amino acid compared to the parent strain, due to a change in the activity of zinc-binding dehydrogenase by introduction of the zinc-binding dehydrogenase variant thereinto. In particular, the amount of L-aromatic amino acid produced by the transformant may be at least 5%, specifically 5 to 60% (preferably 10 to 50%) higher than that produced by the parent strain, and the transformant is capable of producing 4 to 20 g of an L-aromatic amino acid per liter of a culture of the strain.

Still yet another aspect of the present invention provides a method for producing an L-aromatic amino acid, comprising steps of: culturing the transformant in a medium; and recovering an L-aromatic amino acid from the transformant or the medium in which the transformants has been cultured.

The culturing may be performed using a suitable medium and culture conditions known in the art, and any person skilled in the art may easily adjust and use the medium and the culture conditions. Specifically, the medium may be a liquid medium, without being limited thereto. Examples of the culturing method include, but are not limited to, batch culture, continuous culture, fed-batch culture, or a combination thereof.

According to one embodiment of the present invention, the medium should meet the requirements of a specific strain in a proper manner, and may be appropriately modified by a person skilled in the art. For the culture medium for the *Escherichia* sp. strain, reference may be made to, but not limited to, a known document (Manual of Methods for General Bacteriology, American Society for Bacteriology, Washington D.C., USA, 1981).

According to one embodiment of the present invention, the medium may contain various carbon sources, nitrogen sources, and trace element components. Examples of carbon sources that may be used include: sugars and carbohydrates such as glucose, sucrose, lactose, fructose, maltose, starch, and cellulose; oils and fats such as soybean oil, sunflower oil, castor oil, and coconut oil; fatty acids such as palmitic acid, stearic acid, and linoleic acid; alcohols such as glycerol and ethanol; and organic acids such as acetic acid. These substances may be used individually or as a mixture, without being limited thereto. Examples of nitrogen sources that may be used include peptone, yeast extract, meat extract, malt extract, corn steep liquor, soybean meal, urea, or inorganic compounds such as ammonium sulfate, ammonium chloride, ammonium phosphate, ammonium carbonate, and ammonium nitrate. The nitrogen sources may also be used individually or as a mixture, without being limited thereto. Examples of phosphorus sources that may be used include, but are not limited to, potassium dihydrogen phosphate or dipotassium hydrogen phosphate or the corresponding sodium-containing salts. In addition, the culture medium may contain, but is not limited to, metal salts such as magnesium sulfate or iron sulfate, which are required for growth. In addition, the culture medium may contain essential growth substances such as amino acids and vitamins. Moreover, suitable precursors may be used in the culture medium. The medium or individual components may be added to the culture medium batchwise or in a continuous manner by a suitable method during culturing, without being limited thereto.

According to one embodiment of the present invention, the pH of the culture medium may be adjusted by adding compounds such as ammonium hydroxide, potassium hydroxide, ammonia, phosphoric acid and sulfuric acid to the microorganism culture medium in an appropriate manner during the culturing. In addition, during the culturing, foaming may be suppressed using an anti-foaming agent such as a fatty acid polyglycol ester. Additionally, to keep the culture medium in an aerobic condition, oxygen or an oxygen-containing gas (for example, air) may be injected into the culture medium. The temperature of the culture medium may be generally 20°C to 45°C, for example, 25°C to 40°C. The culturing may be continued until a desired amount of a useful substance is produced. For example, the culturing time may be 10 hours to 160 hours.

According to one embodiment of the present invention, in the step of recovering an L-aromatic amino acid from the cultured transformant or the medium in which the transformant has been cultured, the produced L-aromatic amino acid may be collected or recovered from the medium using a suitable method known in the art depending on the culture method. Examples of a method that may be used to recover the produced L-aromatic amino acid include, but are not limited to, centrifugation, filtration, extraction, spraying, drying, evaporation, precipitation, crystallization, electrophoresis, fractional dissolution (e.g., ammonium sulfate precipitation), chromatography (e.g., ion exchange, affinity, hydrophobicity and size exclusion), and the like.

According to one embodiment of the present invention, the step of recovering an L-aromatic amino acid may be performed by centrifuging the culture medium at a low speed to remove biomass and separating the obtained supernatant through ion-exchange chromatography.

According to one embodiment of the present invention, the step of recovering an L-aromatic amino acid may include a process of purifying the L-aromatic amino acid.

According to one embodiment of the present invention, the L-aromatic amino acid may be at least one selected from the group consisting of L-tryptophan, L-phenylalanine, and L-tyrosine.

### Advantageous Effects

The zinc-binding dehydrogenase variant according to the present invention is obtained by substituting one or more amino acids in the amino acid sequence constituting zinc-binding dehydrogenase to change the enzymatic activity of the zinc-binding dehydrogenase, and a recombinant microorganism comprising the zinc-binding dehydrogenase variant is capable of efficiently producing an L-aromatic amino acid.

### Brief Description of Drawings

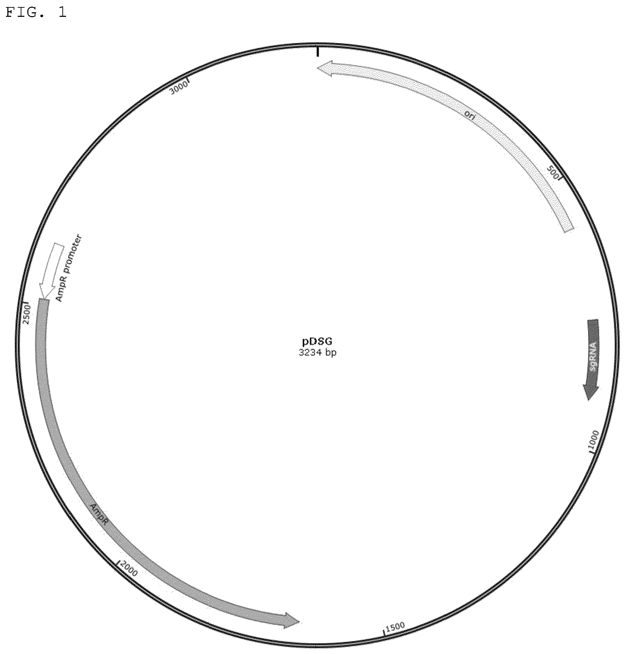

FIG. 1 shows the structure of plasmid pDSG according to one embodiment of the present invention.

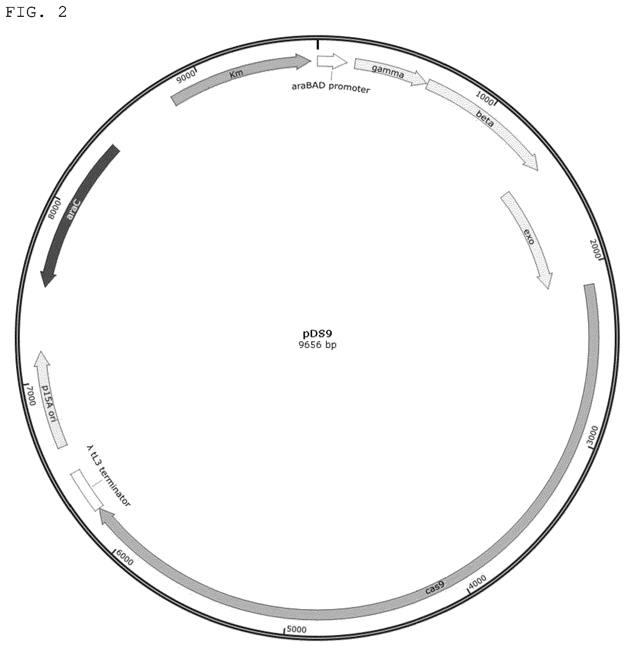

FIG. 2 shows the structure of plasmid pDS9 according to one embodiment of the present invention.

### Mode for Invention

Hereinafter, the present invention will be described in more detail. However, this description is merely presented by way of example to facilitate the understanding of the present invention, and the scope of the present invention is not limited by this exemplary description.

### Example 1. Construction of Strains Expressing Zinc-Binding Dehydrogenase Variant

In order to evaluate the effect of a variant resulting from substitution of arginine for glycine at position 102 of the amino acid sequence of zinc-binding dehydrogenase (SEQ ID NO: 3) on the production of L-aromatic amino acids, a vector expressing the zinc-binding dehydrogenase variant and strains into which the vector has been introduced were constructed. For insertion of the gene encoding the zinc-binding dehydrogenase variant into the strains, plasmids pDSG and pDS9 were used, and the strains were constructed as follows.

Here, the plasmid pDSG has an origin of replication that works only in *Escherichia coli,* contains an ampicillin resistance gene, and has a mechanism for expressing guide RNA (gRNA). The plasmid pDS9 has an origin of replication that works only in *Escherichia coli,* contains a kanamycin resistance gene and λ red genes (exo, bet and gam), and has a mechanism for expressing CAS9 derived from *Streptococcus pyogenes.*

### 1-1. Construction of Vector pDSG-yggP (Gly102Arg) for Transformation

Using *Escherichia coli* MG1655 (KCTC14419BP) gDNA as a template, a fragment upstream of the amino acid mutation at position 102 of the *E. coli* yggP gene encoding zinc-binding dehydrogenase was obtained by PCR using a primer pair of primer 7 and primer 9 and a primer pair of primer 8 and primer 10, and a fragment downstream of the amino acid mutation at position 102 of the *E. coli* yggP gene was obtained by PCR using a primer pair of primer 11 and primer 13 and a primer pair of primer 12 and primer 14. At this time, each of the upstream and downstream fragments contained a sequence that modifies glycine (Gly) at amino acid residue position 102 of the yggP gene into arginine (Arg). In the PCR, Takara PrimeSTAR Max DNA polymerase was used as polymerase, and PCR amplification was performed for 30 cycles under the following conditions: denaturation at 95°C for 10 sec, annealing at 57°C for 15 sec, and polymerization at 72°C for 10 sec.

Using the plasmid pDSG as a template, four pDSG gene fragments were obtained by PCR using a primer pair of primer 3 and primer 5, a primer pair of primer 4 and primer 6, a primer pair of primer 15 and primer 1, and a primer pair of primer 16 and primer 2, respectively. At this time, each of the gene fragments contained a gRNA sequence targeting Gly at position 102 of the yggP gene. The gRNA was selected as a 20 mer upstream of NGG of the sequence to be mutated. In the PCR, Takara PrimeSTAR Max DNA polymerase was used as polymerase, and PCR amplification was performed for 30 cycles under the following conditions: denaturation at 95°C for 10 sec, annealing at 57°C for 15 sec, and polymerization at 72°C for 15 sec.

The obtained fragments upstream and downstream of the amino acid at position 102 of the yggP gene, and the obtained four pDSG gene fragments were cloned using a self-assembly cloning method (BioTechniques 51:55-56 (July 2011)), thereby obtaining a recombinant plasmid which was named pDSG-yggP (Gly102Arg).

### 1-2. Construction of L-Tryptophan- or L-Phenylalanine-Producing Strain into Which Zinc-Binding Dehydrogenase Variant yggP (Gly102Arg) Has Been Introduced

To construct an L-tryptophan-producing strain and an L-phenylalanine-producing strain, *Escherichia coli* KFCC11660P and KCCM10016 were used as parent strains.

The KFCC11660P strain or the KCCM10016 strain was primarily transformed with the plasmid pDS9 and cultured in LB-Km (containing 25 g/L of LB liquid medium and 50 mg/L of kanamycin) solid medium, and then kanamycin-resistant colonies were selected. The selected colonies were secondarily transformed with the pDSG-yggP (Gly102Arg) plasmid and cultured in LB-Amp & Km (containing 25 g/L of LB liquid medium, 100 mg/L of ampicillin and 50 mg/L of kanamycin) solid medium, and then ampicillin- and kanamycin-resistant colonies were selected. Next, a gene fragment was obtained by PCR using a primer pair of primer 17 and primer 18. Here, Takara PrimeSTAR Max DNA polymerase was used as polymerase, and PCR amplification was performed for 30 cycles under the following conditions: denaturation at 95°C for 10 sec, annealing at 57°C for 10 sec, and polymerization at 72°C for 15 sec. The sequence of the gene fragment obtained using the primer pair of primer 17 and primer 18 was analyzed by Macrogen.

The selected secondary transformants were passaged 7 times in LB liquid medium, and colonies were selected on LB solid medium. Each colony was selectively cultured on each of LB, LB-Amp and LB-Km solid media. Colonies that did not grow on the LB-Amp and LB-Km solid media while growing on the LB solid medium were selected. The strains constructed in this way were named KFCC11660P_yggP (Gly102Arg) and KCCM10016_yggP (Gly102Arg), respectively.

The primer sequences used in Example 1 are shown in Table 1 below.

**[Table 1]**

| Primer name | SEQ ID NO. | Primer sequence (5' → 3') |
|---|---|---|
| Primer 1 | 5 | CAATTTTATTATAGTAATTGACTATTATAC |
| Primer 2 | 6 | GCTTGGTAACCAATTTTATTATAGTAATTGACTATTATAC |
| Primer 3 | 7 | GTTACCAAGCGGATATTCAGGTTTTAGAGCTAGAAATAGC |
| Primer 4 | 8 | GGATATTCAGGTTTTAGAGCTAGAAATAGC |
| Primer 5 | 9 | GAGCCTGTCGGCCTACCTGCT |
| Primer 6 | 10 | CGGCCGGCATGAGCCTGTCG |
| Primer 7 | 11 | ATGCCGGCCGGCTGCTATTTATGGCAAGCG |
| Primer 8 | 12 | GCTGCTATTTATGGCAAGCG |
| Primer 9 | 13 | CCCATCGCCGGTTGCAATAC |
| Primer 10 | 14 | GCTTGGTAACCCCATCGCCG |
| Primer 11 | 15 | GTTACCAAGCAGATATTCAGCGGGCTACAG |
| Primer 12 | 16 | AGATATTCAGCGGGCTACAG |
| Primer 13 | 17 | TGCTCATAAACATATTGCGT |
| Primer 14 | 18 | GCCCATGCGATGCTCATAAA |
| Primer 15 | 19 | TCGCATGGGCGAGCTCCTGAAAATCTCGATAAC |
| Primer 16 | 20 | GAGCTCCTGAAAATCTCGATAAC |
| Primer 17 | 21 | ACGTGTGACGGATAAACCAC |
| Primer 18 | 22 | TTCACATACACCAGCTCAAT |

### Experimental Example 1. Evaluation of L-Aromatic Amino Acid Productivity of Mutant Strain into Which Zinc-Binding Dehydrogenase Variant Has Been Introduced

The L-tryptophan or L-phenylalanine productivities of the parent strains (KFCC11660P and KCCM10016) and the mutant strains (KFCC11660P_yggP (Gly102Arg) and KCCM10016_yggP(Gly102Arg)) into which the zinc-binding dehydrogenase variant has been introduced were compared.

1 vol% of each strain (parent strain or mutant strain) was inoculated into a 100-mL flask containing 10 mL of a medium for tryptophan production or a medium for phenylalanine production (the composition of each medium is shown in Table 2 below) and was cultured with shaking at 37°C and 200 rpm for 72 hours. After completion of the culturing, the concentration of L-tryptophan or L-phenylalanine in the medium was measured using HPLC (Agilent), and the results are shown in Tables 3 and 4 below, respectively.

**[Table 2]**

| Medium for tryptophan production | | Medium for phenylalanine production | |
|---|---|---|---|
| Component | Content | Component | Content |
| Glucose | 80.0 g/L | Glucose | 80.0 g/L |
| (NH₄)₂SO₄ | 20.0 g/L | (NH₄)₂SO₄ | 20.0 g/L |
| K₂HPO₄ | 0.8 g/L | K₂HPO₄ | 1.0 g/L |
| K₂SO₄ | 0.4 g/L | KH₂PO₄ | 1.0 g/L |
| MgCl₂ | 0.8 g/L | K₂SO₄ | 0.4 g/L |
| Fumaric acid | 1.0 g/L | MgCl₂ | 1.0 g/L |
| Yeast extract | 1.0 g/L | Fumaric acid | 0.5 g/L |
| (NH₄)₆Mo₇O₂₄ | 0.12 ppm | Yeast extract | 1.0 g/L |
| H₃BO₃ | 0.01 ppm | Glutamic acid | 0.5 g/L |
| CuSO₄ | 0.01 ppm | CaCl₂ | 5.00 ppm |
| MnCl₂ | 2.00 ppm | MnCl₂ | 2.00 ppm |
| ZnSO₄ | 0.01 ppm | ZnSO₄ | 1.00 ppm |
| CoCl₂ | 0.10 ppm | CoCl₂ | 0.10 ppm |
| FeCl₂ | 10.00 ppm | FeCl₂ | 10.00 ppm |
| Thiamine_HCl | 20.00 ppm | Thiamine_HCl | 20.00 ppm |
| L-Tyrosine | 200.00 ppm | L-Tyrosine | 200.00 ppm |
| L-phenylalanine | 300.00 ppm | CaCO₃ | 3% |
| CaCO₃ | 3% | - | - |
| pH 7.0 with NaOH (33%) | | pH 7.0 with NaOH (33%) | |

**[Table 3]**

| Strain | L-tryptophan concentration (g/L) | L-tryptophan concentration increase rate (%) |
|---|---|---|
| KFCC11660P | 4.10 | - |
| KFCC11660P_yggP (Gly102Arg) | 4.87 | 18.7 |

**[Table 4]**

| Strain | L-phenylalanine concentration (g/L) | L-phenylalanine concentration increase rate (%) |
|---|---|---|
| KCCM10016 | 3.42 | - |
| KCCM10016_yggP (Gly102Arg) | 4.21 | 23.1 |

As shown in Tables 3 and 4 above, it was confirmed that the mutant strains KFCC11660P_yggP (Gly102Arg) and KCCM10016_yggP (Gly102Arg) into which the zinc-binding dehydrogenase variant has been introduced showed increases in L-tryptophan production and L-phenylalanine production of about 19% and 23%, respectively, compared to the parent strains KFCC11660P and KCCM10016.

## Claims

1. A zinc-binding dehydrogenase variant consisting of the amino acid sequence of SEQ ID NO: 1 in which glycine at position 102 in the amino acid sequence of SEQ ID NO: 3 is substituted with arginine.

2. A polynucleotide encoding the variant of claim 1.

3. A transformant comprising the variant of claim 1 or the polynucleotide of claim 2.

4. The transformant of claim 3, which is an *Escherichia* sp. strain.

5. The transformant of claim 3, which has ability to produce an L-aromatic amino acid.

6. A method for producing an L-aromatic amino acid, comprising steps of:
culturing the transformant of claim 3 in a medium;
and
recovering an L-aromatic amino acid from the transformant or the medium in which the transformant has been cultured.

7. The method of claim 6, wherein the L-aromatic amino acid is at least one selected from the group consisting of L-tryptophan, L-phenylalanine, and L-tyrosine.

## Patentansprüche

1. Zinkbindende Dehydrogenase-Variante, bestehend aus der Aminosäuresequenz unter SEQ ID NO: 1, in der Glycin an Position 102 in der Aminosäuresequenz unter SEQ ID NO: 3 durch Arginin substituiert ist.

2. Polynukleotid, codierend die Variante nach Anspruch 1.

3. Transformante, umfassend die Variante nach Anspruch 1 oder das Polynukleotid nach Anspruch 2.

4. Transformante nach Anspruch 3, bei der es sich um einen Escherichia sp.-Stamm handelt.

5. Transformante nach Anspruch 3, die in der Lage ist, eine aromatische L-Aminosäure zu produzieren.

6. Verfahren zur Herstellung einer aromatischen L-Aminosäure, welches die folgenden Schritte umfasst:
Kultivieren der Transformante nach Anspruch 3 in einem Medium und
Gewinnen einer aromatischen L-Aminosäure aus der Transformante oder dem Medium, in dem die Transformante kultiviert wurde.

7. Verfahren nach Anspruch 6, wobei die aromatische L-Aminosäure mindestens eine aus der aus L-Tryptophan, L-Phenylalanin und L-Tyrosin bestehenden Gruppe ausgewählte ist.

## Revendications

1. Variant de déshydrogénase liant le zinc constitué de la séquence d'acides aminés de SEQ ID NO: 1 dans laquelle la glycine en position 102 dans la séquence d'acides aminés de SEQ ID NO: 3 est remplacée par l'arginine.

2. Polynucléotide codant pour le variant de la revendication 1.

3. Transformant comprenant le variant de la revendication 1 ou le polynucléotide de la revendication 2.

4. Transformant selon la revendication 3, qui est une souche de Escherichia sp..

5. Transformant selon la revendication 3, qui a la capacité de produire un L-aminoacide aromatique.

6. Procédé de production d'un L-aminoacide aromatique, comprenant les étapes de :
culture du transformant de la revendication 3 dans un milieu ; et
récupération d'un L-aminoacide aromatique à partir du transformant ou du milieu dans lequel le transformant a été cultivé.

7. Procédé selon la revendication 6, le L-aminoacide aromatique étant au moins l'un choisi dans le groupe constitué par le L-tryptophane, la L-phénylalanine et la L-tyrosine.
